# EUROPEAN PATENT SPECIFICATION

(11) **EP 3 406 738 B1**
(45) Date of publication and mention of the grant of the patent: **09.12.2020**
(21) Application number: 18173646.3
(22) Date of filing: 22.05.2018
(51) Int. Cl.: C12Q 1/6895

(54) **METHOD FOR DETECTING MACROPHOMINA PHASEOLINA**
VERFAHREN ZUM NACHWEISS VON MACROPHOMINA PHASEOLINA
MÉTHODE POUR DETECTER MACROPHOMINA PHASEOLINA

(30) Priority: 26.05.2017 IT 201700057466
(43) Date of publication of application: 28.11.2018
(73) Proprietor: Università di Pisa, 56126 Pisa (IT)
(72) Inventor: DA LIO, Daniele, 50026 San Casciano in Val di Pesa Firenze (IT); PECCHIA, Susanna, 57023 Cecina Livorno (IT)
(74) Representative: Biggi, Cristina

(56) References cited:
- BANDAMARAVURI KISHORE BABU ET AL: "Identification and detection of Macrophomina phaseolina by using species-specific oligonucleotide primers and probe", MYCOLOGIA., vol. 99, no. 6, 1 November 2007 (2007-11-01), pages 797-803, XP055453052, ISSN: 0027-5514, DOI: 10.1080/15572536.2007.11832511
- B N Chakraborty ET AL: "rDNA Sequence and Phylogenetic Analysis of Macrophomina phaseolina, Root Rot Pathogen of Citrus reticulata (Blanco)", Global Journal of Molecular Sciences, 1 January 2011 (2011-01-01), pages 26-34, XP055453281, Retrieved from the Internet: URL:https://idosi.org/gjms/gjms6(2)/1.pdf [retrieved on 2018-02-22]
- ALI N. KHAN ET AL: "Molecular Identification and Genetic Characterization of Macrophomina phaseolina Strains Causing Pathogenicity on Sunflower and Chickpea", FRONTIERS IN MICROBIOLOGY, vol. 8, 19 July 2017 (2017-07-19), XP055453063, DOI: 10.3389/fmicb.2017.01309
- D. DA LIO ET AL: "DEVELOPMENT OF A RAPID PCR NUCLEIC ACID LATERAL FLOW IMMUNOASSAY (PCR-NALFIA) BASED ON rDNA IGS SEQUENCE ANALYSIS FOR THE DETECTION OF MACROPHOMINA PHASEOLINA IN SOILS", JOURNAL OF PLANT PATHOLOGY, vol. 99, 22 September 2017 (2017-09-22), pages s44-s45, XP055453361, DOI: 10.4454/jpp.v99i1SUP.3946

### SEQUENCE LISTING

<110> Università di Pisa
<120> Method for determining Macrophomina phaseolina
<130> 21.U0053.12.EP.4
<160> 8
<170> PatentIn version 3.5
<210> 1
   <211> 891
   <212> DNA
   <213> Artificial Sequence
<220>
   <223> 891 IGS Region M. phaseolina
<400> 1
   ccgctccgcc atacaaatta ttttttcgaa aaaatcaatg cgcgcgcgcg aacatttttc 60
   tgaaaaatty gggctacacg agggtcggyg ggtatatgag aagaagagag cgaaggcaat 120
   ctggaaraga gtgaaagacc tctattccag agctgctcga aggtctcttc tcytacgagc 180
   cgtaaagcgg cgccggtcct caggctgtaa ctggcctttg gcggtgagcc gttttgcagc 240
   caccygctaa cgtgggtggt agtcccgtac ctgcggagcc ggcttccaaa ggtcttgcag 300
   acggcggtgc aacgcaccgt acgagcgggc cttggtgctg gtttcatgcg gcggacttgg 360
   ggcccgcgtt ctcgcccttc acggggcggt ccgcgtcccg tcaaccgcac ctgaagcccc 420
   gctggctagt tctagcgggc gtggatggag tctgtggatg gttggccgat ggctaccatt 480
   tttcacaaga cactcggggg taagcccaag ccggtttccg acgtctgaaa ggacgtcgac 540
   gcccggtgaa cggcatcctc ctcctgaggc gtgcatgtcg cagttcgttt tgttgcgccc 600
   ctaaccgggc ccggacgttt ctgcggcgga ccacgccttg gggagcttag tgggggcaat 660
   cggatggcct tgaagggccc tcccgcaggt gctaacgcgc cgcggrttgg gacccctgag 720
   gtcgagcgct ctttcgggcg ctccttcctt ctaactaccc tgcctccttc gggaggtttt 780
   gtcccggggc cctytagcgg cgcggtcctt cggggcgtgc gcggcgacgg gggaacyggg 840
   acgctaggat agttacctgg ttgattctgc cagtagtcat atgcttgtct c 891
<210> 2
   <211> 460
   <212> DNA
   <213> Artificial Sequence
<220>
   <223> 460 IGS Region M. phaseolina
<400> 2
   gctaacgtgg gtggtagtcc cgtacctgcg gagccggctt ccaaaggtct tgcagacggc 60
   ggtgcaacgc accgtacgag cgggccttgg tgctggtttc atgcggcgga cttggggccc 120
   gcgttctcgc ccttcacggg gcggtccgcg tcccgtcaac cgcacctgaa gccccgctgg 180
   ctagttctag cgggcgtgga tggagtctgt ggatggttgg ccgatggcta ccatttttca 240
   caagacactc gggggtaagc ccaagccggt ttccgacgtc tgaaaggacg tcgacgcccg 300
   gtgaacggca tcctcctcct gaggcgtgca tgtcgcagtt cgttttgttg cgcccctaac 360
   cgggcccgga cgtttctgcg gcggaccacg ccttggggag cttagtgggg gcaatcggat 420
   ggccttgaag ggccctcccg caggtgctaa cgcgccgcgg 460
<210> 3
   <211> 102
   <212> DNA
   <213> Artificial Sequence
<220>
   <223> 102 Region IGS M. phaseolina
<400> 3
   ggtagtcccg tacctgcgga gccggcttcc aaaggtcttg cagacggcgg tgcaacgcac 60
   cgtacgagcg ggccttggtg ctggtttcat gcggcggact tg 102
<210> 4
   <211> 18
   <212> DNA
   <213> Artificial Sequence
<220>
   <223> MP102F
<400> 4
   ggtagtcccg tacctgcg 18
<210> 5
   <211> 19
   <212> DNA
   <213> Artificial Sequence
<220>
   <223> MP102R
<400> 5
   caagtccgcc gcatgaaac 19
<210> 6
   <211> 19
   <212> DNA
   <213> Artificial Sequence
<220>
   <223> MP1F
<400> 6
   gattccctcc catcctccc 19
<210> 7
   <211> 20
   <212> DNA
   <213> Artificial Sequence
<220>
   <223> MP1R
<400> 7
   cctcgtgtag cccgaatttt 20
<210> 8
   <211> 20
   <212> DNA
   <213> Artificial Sequence
<220>
   <223> MP891F
<400> 8
   ccgctccgcc atacaaatta 20

## Description

### TECHNICAL FIELD OF THE INVENTION

The present invention relates to a method for determining the presence of the phytopathogenic fungus *Macrophomina phaseolina* in a biological sample of interest. The present invention further relates to a kit for implementing the method of the invention.

### PRIOR ART

*Macrophomina phaseolina* (*M. phaseolina*) is a pathogenic fungus belonging to the family *Botryosphaeriaceae,* with a generally telluric *habitus*, capable of infecting over 500 cultivated and spontaneous plant species. It is considered a "global pathogen".

There are multiple symptoms of infection on plants, in particular, for example, wilting of young plants, charcoal rot, dry root rot, leaf and stem wilt, pre- and post-emergence mortality of young plants and stem and root rot.

The development of disease is favoured by high temperatures (the optimal temperature for growth of the fungus is 30-35°C) followed by water stress, though in some cases (jute, maize) the pathogen prefers hot-humid conditions for its development.

A considerable increase in the incidence and severity of *M. phaseolina* infections has recently been observed in many parts of the world, above all in arid environments, a phenomenon that may be attributed to the global warming underway (Chakraborty, 2005; Burdon *et al.,* 2006; Garrett *et al.,* 2006; Crowl *et al.,* 2008; Eastburn *et al.,* 2011). Furthermore, it has been hypothesised that with increases in temperatures *M. phaseolina* could also spread in central and northern Europe, with a 25-35% increase in pervasiveness.

Under unfavourable environmental conditions, the pathogen can remain viable for 2-15 years in soil and/or in infected crop residues in the form of microsclerotia, vegetative structures designed to preserve the fungus and which act as a primary source of inoculation (Meyer *et al*., 1973; Papavizas, 1977; Short *et al.,* 1980; Baird *et al.,* 2003). When the environmental conditions become favourable, the microsclerotia germinate, emitting hyphae which infect the roots and/or stems of the host plant by penetrating into cells, both by mechanical pressure and through the use of enzymes that degrade the cell wall of the host.

Control of the disease is particularly difficult, as microsclerotia are structures with rather thick, highly melanised walls and they are thus resistant both to adverse environmental conditions and physical and/or chemical treatments.

It is evident, therefore, that being able to identify and quantify the presence of *M. phaseolina* in soil is of fundamental importance in order to seek to contain and limit the infection.

Various microbiological methods for isolating and quantifying M. *phaseolina* from soil are known in the prior art (for example, Watanabe *et al.,* 1970, McCain and Smith, 1972; Meyer *et al.,* 1973; Papavizas and Klag, 1975; Mihail and Alcom, 1982; Cloud and Rupe, 1991). However, such methods have several disadvantages, including, for example, the fact that they require a great deal of laboratory space, are not sufficiently sensitive and/or selective, require a great deal of work and time both for preparation and to obtain the result, depend on the type of soil analysed and, in many cases, require the use of highly toxic chemical substances. The fact that *M. phaseolina* is a weak competitor when cultured in Petri plates together with other fungi present in the soil contributes to complicating the methods further (Cook *et al.,* 1973). In fact, it has been observed that the simultaneous development of other fungi considerably reduces the growth of *M. phaseolina* and/or inhibits the production of microsclerotia, on which correct identification of the pathogen depends.

In order to overcome the above-mentioned difficulties tied to the determination of the presence of *M. phaseolina* in soil with methods of a microbiological type, it is possible to rely on diagnostic techniques based on nucleic acids, which are more rapid and sensitive in identifying pathogenic fungi.

In the case of *M. phaseolina,* a molecular method has recently been developed which makes it possible to determine the presence of the fungus in infected plants or in rhizosphere soil by amplification of a portion of the SCAR (Sequenced Characterized Amplified Region) by real-time PCR (Babu *et a*/*.,* 2011). However, this method uses primers characterised by 100% similarity for all species taxonomically close to M. *phaseolina* and the method thus has low specificity and moreover, as it makes use of real-time PCR, it is not a simple, practical and economical method.

The technical problem that the present invention aims to overcome is that of developing, and hence providing, a method for determining the presence of *M. phaseolina* in a biological sample that overcomes the limits and drawbacks of the known methods of the prior art commonly used up to now.

The present invention solves the aforesaid technical problem by means of a method that comprises amplifying specific regions of *M. phaseolina* which distinguish this fungus from species phylogenetically very close to it. The method advantageously enables the determination of the presence of *M. phaseolina* or in any case the identification or typing thereof in a biological sample in a more specific and selective manner compared to the methods presently available for that purpose. In particular, the method is highly advantageous because it enables the identification of as little as one microsclerotium per gram of soil, which is a very relevant aspect considering that even a single microsclerotium g⁻¹ soil is capable of causing disease. In addition, the method is also advantageously simple, rapid and does not require special and/or costly equipment or instruments, nor does it entail the use of chemical substances and/or physical agents that are toxic or hazardous for the operator and it can be implemented directly on site.

The method of the present invention is described below in greater detail, also using examples and the figures, which have no limiting purpose.

### DETAILED DESCRIPTION

A first aspect of the present disclosure relates to a method for determining the presence of *M. phaseolina,* preferably for detecting the presence of the DNA thereof and/or as little as one microsclerotium per gram of soil, in a biological sample, comprising the steps of:
(i) Obtaining a biological sample suspected for the presence of M. *phaseolina,* possibly in the form of a microsclerotium, preferably a biological sample suspected for the presence of DNA of *M. phaseolina;*
(ii) Amplifying at least one portion of SEQ ID NO: 1 with a pair of oligonucleotides (primers); and
(iii) Detecting the amplified sequence (also called amplicon), if present, by means of an analytical method wherein the presence of said amplified sequence signifies the positivity of the sample for the presence of M. *phaseolina*; it preferably signifies the positivity of the sample for the presence of the DNA of *M. phaseolina,* i.e. that the sample which underwent the method is *M. phaseolina-positive,* that is, contaminated by *M. phaseolina,* preferably contaminated by the DNA of *M. phaseolina.*

SEQ ID NO: 1 corresponds to a sequence of 891 base pairs (bp) that falls in the 3'-terminal IGS region of the ribosomal DNA (rDNA) of M. *phaseolina,* wherein the ambiguous bases have been considered in the consensus using the code of the organisation IUPAC (e.g. R, Y, W, etc.). In the context of the present invention, microsclerotium means a fungal structure of small dimensions, produced for the purpose of withstanding unfavourable environmental conditions and made up of aggregates of melanised hyphae.

In the context of the present invention, IGS is an acronym of intergenic spacer and refers to a spacer present within the region of the ribosomal DNA that comprises the 18S, 5.8S and 28S genes (i.e. the genes coding for the 18S, 5.8S and 28S rRNA, which are the subunits of the ribosomes), the latter separated by internal transcribed spacers called ITS1 and ITS2 and an intergenic spacer IGS. The IGS region is almost entirely untranscribed and thus highly variable. Furthermore, it is a very large region - usually about 2000-5000bp long - and it is very rich in GC content (i.e. repetitions of the nucleotides guanine and cytosine) and therefore rather difficult to sequence.

In the context of the present invention, ambiguous bases means nonspecific nucleotides that provide possible nucleotide combinations present in a genomic sequence, i.e. a single ambiguous nucleotide can represent from two to four different unambiguous nucleotides.

In the context of the present invention, consensus sequence means a nucleotide sequence containing the most frequent nucleotides for each position obtained through an alignment of sequences.

According to the present invention, step (ii) comprises amplifying at least a portion of SEQ ID NO: 3 with a pair of oligonucleotides (primers).

SEQ ID NO: 2 is a sequence 460 base pairs long whilst SEQ ID NO: 3 is a sequence 102 base pairs long.

SEQ ID NO: 2 and 3 are included in SEQ ID NO: 1 and SEQ ID NO: 3 is also included in SEQ ID NO: 2.

In a preferred embodiment of the invention, the pair of primers used for the amplification step (ii) are SEQ ID NO: 4 and 5 (respectively indicated in the example as MP102F (forward) and MP102R (reverse)). However, any pair of primers usable for amplifying at least a portion of SEQ ID NO: 1 and/or SEQ ID NO: 2 and/or SEQ ID NO: 3 must be considered as covered by the present disclosure.

SEQ ID NO: 1-3 can be used as a positive control of the method, or the genomic DNA of *M. phaseolina* or a sample comprising said DNA may be used as a positive control.

The pair of primers of step (ii), preferably SEQ ID NO: 4 and 5, enable the amplification of a region of the DNA of *M. phaseolina* that is extremely specific for this fungus. Consequently, the method based on the use of these primers is highly selective and enables the presence of genetic material of this fungus to be identified in an extremely sensitive manner. Therefore, the method of the present invention serves to identify the presence of *M. phaseolina* or of the DNA thereof or in any case to type *M*. *phaseolina.*

The sequences used in the present disclosure are set forth in the table below and in the sequence listing accompanying this application. Sequences having 80-99% identity with the sequences disclosed here below must be considered as part of the subject matter of the present disclosure.

**Table I**

| | | |
|---|---|---|
| SEQ ID NO: 1 | | Region 891 IGS *M. phaseolina* |
| SEQ ID NO: 2 | | Region 460 IGS *M. phaseolina* |
| | | |
| SEQ ID NO: 3 | | Region 102 IGS *M. phaseolina* |
| SEQ ID NO: 4 | GGTAGTCCCGTACCTGCG | MP102F |
| SEQ ID NO: 5 | CAAGTCCGCCGCATGAAAC | MP102R |

According to a preferred aspect of the present invention, the biological sample can be soil, an infected plant tissue, preferably selected from among: leaves, stalk, roots, fruits, or seeds, and potentially infected crop residues.

The sample is preferably a soil sample suspected for the presence of M. *phaseolina.*

The *M. phaseolina,* if any, can be present in the sample as mycelium or in the form of microsclerotia.

According to a preferred aspect of the present invention, the DNA of the fungus according to step (i) of the method, if present, is extracted from the sample with any of the techniques known to the person skilled in the art which are usable for the designated purpose. In particular, the extraction method (isolation, purification) will depend on the type of biological sample undergoing the method of the invention.

In particular, the authors of the present invention have developed a purification method that enables nucleic acids, such as DNA, to be extracted from soil samples. Any type of soil is suited to the method described below.

The method developed is clearly valid for isolating DNA, or also other nucleic acids, of any microorganism present in the soil, i.e. bacteria and/or fungi and/or yeasts.

In this regard, we note that soil is a very complex ecosystem from both a physiochemical and microbiological standpoint and when the nucleic acids extracted from soil samples are analysed, usually by PCR, the main problem is represented by the loss and/or degradation thereof due to the absorption of the nucleic acids by the soil particles themselves and by the presence of compounds that inhibit the PCR reaction.

Therefore, when the biological sample undergoing the method is soil, the technique of isolating the nucleic acid from said sample is critical for the success of the method, in particular when it is aimed at determining any microorganisms contained therein, especially if pathogens.

In fact, during the process of purification of the nucleic acid the coextraction of organic substances (humic acids, fulvic acids, cellular debris, proteins, solvents, pesticides) and/or inorganic ones (heavy metals, clay minerals) may occur, which can compromise the subsequent steps. For example, it is known that humic acids - whose presence is revealed by the brown colour that the extracted nucleic acid exhibits - are capable of impeding PCR, since they chelate Mg²⁺ ions, consequently inhibiting Taq DNA polymerase (Tsai *et al.,* 1992; Tebbe and Vahjen, 1993; Harry *et al.,* 1999; Miller *et al.,* 1999; Roose-Amsaleg *et al.,* 2001; Takada-Hoshino and Matsumoto, 2004). There are scientific publications that describe the possibility of solving this problem by performing a dilution of the extracted DNA, or by adding agents capable of sequestering the inhibitors, such as bovine serum albumin (BSA) to the reaction mixture (Tebbe and Vahjen, 1993; Yeates *et al.,* 1997). In many cases, however, even a very high dilution (1000 times) is not able to eliminate the PCR inhibition effect of the coextracted humic acids (Erb and Wagner-Döbler, 1993).

The most widely applied techniques for the purification of nucleic acids from soil samples are generally based on ultracentrifugation in a cesium chloride density gradient, ion exchange chromatography, agarose gel electrophoresis or dialysis (Roose-Amsaleg *et al.,* 2001). In recent years, many protocols for the extraction of nucleic acids, in particular DNA, from soil samples have been developed which generally provide for cell lysis, subsequent removal of cell fragments and deproteination and, finally, precipitation of the nucleic acid. Clearly, the purified nucleic acid can also undergo further purification steps.

The step of cell lysis can take place directly in the soil, and in such a case one speaks of direct extraction. Alternatively, any microorganisms included in the soil sample are isolated and then lysis is performed and in this case one speaks of indirect extraction. In any case, lysis can take place chemically, enzymatically or mechanically. The first two methods are more delicate and thus do not completely compromise the integrity of the nucleic acid. However, they are often ineffective against some cells and/or do not thoroughly penetrate among the soil particles.

Combinations of detergents and lytic enzymes are generally used for lysis. For example, sodium dodecyl sulphate (SDS), used hot or cold to limit the coextraction of humic acids (Volossiouk *et al.,* 1995; Zhou *et a*/*.,* 1996). Lytic enzymes include lysozyme, capable of hydrolysing the polysaccharides making up the bacterial cell wall and some proteases such as proteinase K, achromopeptidase and pronase, which can help to free the nucleic acids (Tebbe *et a*/*.,* 1993; Zhou *et a*/*.,* 1996; Niemi *et a*/*.,* 2001). Furthermore, the use of polyvinylpolypyrrolidone (PVPP) and hexadecyltrimethylammonium bromide (CTAB), used individually or mixed in the extraction buffer, can improve the purity of the nucleic acid obtained, since these substances complex the humic acids (Zhou *et al.,* 1996; Porteous *et al.,* 1997). Mechanical lysis succeeds in breaking up the soil particles and disrupting a large part of the cells present, but often produces degraded genetic material. Mechanical lysis can take place by grinding with a mortar and pestle, for example in liquid nitrogen, thermal shock (freezing-thawing), homogenisation with a bead-mill or bead-beating, heating with microwaves, ultrasonication, etc. The bead-beating method has revealed to be better than both thermal shock and chemical lysis, although the extracted molecules of DNA are smaller (Cullen and Hirsch, 1998; Miller *et al.,* 1999). Mechanical and chemical methods of lysis are generally combined, taking into consideration the properties of the soil, in particular the content of organic substance and clay minerals, as well as the characteristics of the microorganisms in question. After cell lysis, the freed proteins are usually removed with organic solvents (phenol, chloroform, isoamyl alcohol) used individually or in a mixture (Tebbe *et al.,* 1993; Zhou *et al.,* 1996); deproteination can also be achieved with saline solutions containing sodium chloride, potassium chloride, ammonium acetate, potassium acetate or sodium acetate (Roose-Amsaleg *et al.,* 2001). Once the proteins have been removed, the nucleic acids are precipitated using ethanol, isopropanol or polyethylene glycol (PEG). Alcohol precipitation seems to give low yields and favour the coextraction of humic acids, whereas the use of PEG gives good yields but entails a further step of purification with phenol. Isopropanol precipitation seems to reach the right compromise between DNA recovery yield and extract purity (Zhou *et al.,* 1996; Porteous *et al.,* 1997).

There also exist commercial kits for directly extracting nucleic acids, such as DNA, from soil. Some are based on the lysis of soil microorganisms by combining heat, detergents and a mechanical action (by means of specific beads) using a vortex and the DNA released is then purified by means of a spin filter. Other kits, by contrast, achieve a mechanical lysis with silica and ceramic beads in a bead-beater. Finally, a silica matrix purifies the extracted genomic DNA. The material obtained with such kits is usually usable for PCR without the need for further purification steps.

The authors of the present invention have developed a method for the isolation (purification/extraction) of nucleic acids from soil which, surprisingly, shows numerous advantages compared to the methods known up to now and described above.

The method comprises at least the following steps:
(i) providing a soil sample (soil); and/or
(ii) adding to the soil sample of step (i) a homogenisation buffer comprising polyvinylpyrrolidone (PVP40) and a suspension of milk, preferably skimmed, in sterile water; and/or
(iii) homogenising the suspension obtained from step (ii), preferably by means of a homogeniser of the bead-beater type.

The method preferably comprises the following steps:
(i) providing a soil sample (soil), which has preferably been dried, preferably in air, and reduced into particles and/or sieved, the particles preferably have a size smaller than 4 mm, preferably 2 mm; and/or
(ii) adding to the soil sample obtained from step (i) a homogenisation buffer comprising polyvinylpyrrolidone (PVP40) and a suspension of milk, preferably skimmed, in sterile water; and/or
(iii) homogenising the suspension obtained from step (ii), preferably by means of a homogeniser of the bead-beater type; and/or
(iv) centrifuging, preferably at about 12000 × g for about 20-30 minutes, preferably cold, to remove the soil and any other debris and collect the supernatant (the non-solid part); and/or
(v) lysing the supernatant with a solution preferably comprising proteinase K at a concentration of about 30-100 □g/ml and a 1-5%, preferably about 2%, solution of SDS; and/or
(vi) centrifuging, at about 2500 × g per 7-15 minutes, preferably cold, to remove the cellular debris and collect the supernatant; and/or
(vii) treating the supernatant collected from step (vi) with 1/10 volume of a solution comprising CTAB, preferably after having brought the concentration of NaCl to about 1.4 M; and/or
(viii) precipitating the proteins, preferably with a solution that comprises a mixture of chloroform and isoamyl alcohol; and/or
(ix) centrifuging and collecting the aqueous phase; and/or
(x) precipitating the nucleic acids, preferably with an alcohol, preferably selected from among: ethanol, isopropanol, polyethylene glycol and combinations thereof.

Step (ii) is preferably carried out in the presence of a mixing means, such as meshes/beads, preferably glass, which have a diameter preferably ranging from 0.1 mm to 1 cm, preferably 0.3-0.6 mm.

The milk is preferably skimmed milk powder, such as Transilat 2 by Plasmon, present in a concentration ranging from 0.5 to 10%, preferably 2.5 to 5%, more preferably it is about 3-3.2%.

The use of polyvinylpyrrolidone (PVP40) and skimmed milk during the homogenisation step advantageously makes it possible to avoid co-extracting, together with the nucleic acids, organic substances (humic acids, fulvic acids, cellular debris, proteins, solvents, pesticides) and/or inorganic ones (heavy metals, clay minerals) which may compromise the subsequent uses of the nucleic acids. Consequently, the method of the present invention avoids the further steps of nucleic acid purification, typically based on ultracentrifugation in a cesium chloride density gradient, ion exchange chromatography, agarose gel electrophoresis or dialysis, which are laborious and/or costly.

Homogenisation is carried out at least once, preferably 3-5 times for about 60 seconds at 2000-5000 rpm, preferably about 4000 rpm. Between one step and another, the sample is placed at a low temperature, preferably under ice, for a time of about 60 seconds.

Furthermore, the use of glass beads and/or a bead-beater to homogenise the samples makes it possible to avoid the use of more complex and inconvenient systems such as liquid nitrogen, a mortar, thermal shocks, microwaves, ultrasonication, etc.

Finally, the speed of the homogenisation step with this system enables many samples to be processed simultaneously.

Step (v) and/or (vii) preferably comprise an incubation of the sample at about 60-70°C, preferably about 65°C, for a time of about 30-50 minutes, preferably about 40 minutes for step v and/or about 10-20 minutes, preferably about 15 minutes, for step vii. In the latter case a cooling step may follow for a few minutes, preferably about 2 minutes at 10-20°C, preferably at about 15°C.

In short, the method developed by the authors enables savings in operative terms and in terms of time, money and the consumption of materials and instruments.

The analyses by gel electrophoresis and spectrophotometric quantification of the DNA extracted from agricultural soil with the above-described method in fact demonstrated a good integrity of the DNA as well as a good degree of purification (A260/280: 1,5-1,9). Furthermore, the concentrations obtained were in the order of 50-150 ng µl⁻¹.

Therefore, according to a preferred embodiment, the biological sample tested with the method of the present invention, when it is soil, can undergo the above-described nucleic acid extraction method before being amplified (i.e. before the amplification step (ii)).

The detection step of the method of the invention is carried out with the common analytical methods known to the person skilled in the art for that purpose, for example agarose gel electrophoresis, which has the disadvantage, however, of using delicate and costly instruments, requiring a certain amount of time for its implementation and using substances that are hazardous for the operator.

According to a preferred embodiment of the invention, the detection step of the method of the invention is carried out using a more practical, quicker and safer technique for the detection of amplified sequences based on the use of an immunochromatographic assay of the nucleic acids. More preferably, said detection is achieved by means of an immunochromatographic assay of the nucleic acids with a lateral flow device, or NALFIA, nucleic acid lateral flow immunoassay.

The use of NALFIA for the detection of specific PCR products is known in the prior art (Rule *et a*/*.,* 1996) and it is widely used, mainly as a point-of-care (POC) diagnostic tool in the medical field, as well as for monitoring foods and environmental samples and the analysis of GMOs (Posthuma-Trumpie *et al.,* 2009; Ngom *et al.,* 2010; O'Farrel, 2013).

In the NALFIA assay, the nucleic acids are "captured" on a lateral flow device (LFD), commonly called a strip, which incorporates the reagents (ligands) for carrying out the assay.

The detection of specific PCR products is possible thanks to the use, while carrying out the PCR itself, of primers marked with two different molecules. One primer is generally marked with biotin, the other with fluorescein isothiocyanate (FITC). Possible markers are: fluorescein isothiocyanate (FITC), biotin, digoxigenin (DIG), Texas Red® or dinitrophenol (DNP).

As is well known, a typical strip comprises a "sample pad" where the sample is deposited, a "conjugate pad" in which the tracer is adsorbed, a membrane where the binding reaction of the assay takes place and in which there are two active sites, one for the test and a control site, and, finally, an "absorbent pad" responsible for directing flow through the membrane. The role of the tracer is to provide a signal, not only at the test site (in the case of a positive result), but also at the control site of the strip, to indicate the correct execution of the assay.

When the sample is loaded onto the sample pad, it moves by capillary action towards the conjugate pad where it solubilises the tracer. The tracer is generally an anti-analyte antibody (anti-FITC in the typical case described above), preferably adsorbed to colloidal gold particles. The sample then moves through the membrane towards the zones where the anhydride reagents for the test are deposited.

In the test zone there is a ligand capable of interacting with one of the molecules with which the primers were marked, for example avidin (which binds biotin). In the control zone, by contrast, there are capture antibodies that bind the tracer irrespective of the concentration of the analyte in the sample. If the control zone is not coloured at the end of the assay, the test is to be considered invalid.

When the sample does not contain the amplification product, or amplicon, of interest (using the marked primers), there will be no interaction between the marker and ligand in the test zone of the membrane and therefore no colour will appear. However, if the amplicon of interest is present in the sample, an analyte - antibody - colloidal gold complex is formed, which is recognised by the ligand in the test zone and precipitates there, forming a coloured band of an intensity proportional to the concentration of analyte in the sample.

The main advantage of the lateral flow devices such as in the NALFIA assay is the fact the tests are rapid, simple and economical, as they do not require sophisticated tools or costly reagents; in particular, they make it possible to avoid real-time PCR and electrophoresis, and thus avoid the use of hazardous agents and/or substances such as UV rays and ethidium bromide. Furthermore, thanks to the absence of enzymes or other liquid bio-reagents, lateral flow devices have high stability and a long shelf life. Moreover, the results obtained with such devices are scarcely sensitive to temperature variations, since they are not determined by enzymatic activities and take place in shorter periods of time than assays that use the latter. The test and control lines are clearly visible to the naked eye, thus ensuring the immediacy of the result.

In one embodiment, immunochromatographic nucleic acid assays such as NALFIA can be used in step (iii) of the method according to the invention to detect the presence of *M. phaseolina,* preferably of the DNA thereof, preferably extracted from mycelium and/or microsclerotia cultures. In another embodiment, the above-mentioned immunochromatographic assays can be used to detect the presence of *M. phaseolina* or the DNA thereof extracted directly from the soil that is supposed to be infected by the fungus.

In particular, the authors of the present invention have observed that the NALFIA assay has proven to be much more sensitive in detecting the marked amplicons (PCR products) than the common agarose gel electrophoresis techniques, both with DNA extracted from microsclerotia (without any soil contaminant), and DNA extracted from microsclerotia inoculated into the soil. In the experiments carried out, the NALFIA assay was capable of identifying an amount of PCR product equal to barely 1.92 fg.

If an assay of an immunochromatographic type is employed in step (iii) of the method of the invention, the primers used to carry out the PCR in step (ii) must be marked with markers recognised by the ligands present in the detection device. Typically, it is possible to use fluorescein isothiocyanate (FITC) and/or biotin as markers.

Another aspect of the invention relates to SEQ ID NO: 3-5 and its use in the determination of *M. phaseolina* or the genome thereof, or the genomic DNA thereof.

In particular, SEQ ID NO: 4 and 5 are the primers that enable the amplification of a genomic region of about 102 bp that is located within the IGS region of the ribosomal DNA of *M. phaseolina.* This sequence is extremely specific for the fungus *M. phaseolina* and, as noted, a positive result of an amplification that uses SEQ ID NO: 4 and 5 in particular is correlated to the presence of this fungal pathogen in the examined sample.

In one embodiment of the invention, the primers can be marked at the 5' and/or 3' end. The primers are marked with different markers, preferably at the 5' end. The marker can be any detectable marker; the marker is preferably preselected from among: fluorescein isothiocyanate (FITC) and biotin.

In one embodiment, one primer (preferably the reverse primer) is marked, preferably at the 5' end, with FITC and the other primer (preferably the forward primer) is marked, preferably at the 5' end, with biotin.

In another embodiment, the primers can be lyophilised.

A further aspect of the present invention relates to a kit for determining the presence of *M. phaseolina* or the DNA thereof (i.e. for identifying *M*. *phaseolina),* characterised in that it comprises at least one pair of primers for amplifying at least one portion of a specific *M. phaseolina* sequence, the sequence being selected from among: SEQ ID NO: 3-5.

Said primers of the kit are preferably marked at their end, preferably at the 5' end. The marker is preferably selected from among: fluorescein isothiocyanate (FITC) and biotin.

In one embodiment, a primer (preferably the reverse primer) is marked, preferably at the 5' end, with FITC and the other primer (preferably the forward primer) is marked, preferably at the 5' end, with biotin.

In another embodiment, the primers are lyophilised.

In one embodiment of the invention, the kit further comprises reagents generally used for carrying out PCR.

In one embodiment, both the PCR reagents and the primers can be in the reaction tube of the kit.

In another embodiment, the PCR reagents can be in the reaction tube of the kit whilst the primers can be stored in a separate container and added to the reaction tube only at the time it is desired to carry out the PCR. According to a further preferred embodiment of the invention, the kit also comprises strips or lateral flow devices to enable POC detection by NALFIA. Each strip incorporates the reagents (ligands) for performing the assay as described above.

An example embodiment of the method according to the invention for identifying of the pathogenic fungus *M. phaseolina* is given below; this example is obviously not to be construed as limiting the scope of the invention.

### EXAMPLE

### - Extraction of nucleic acids from soil contaminated by M. phaseolina

A soil sample contaminated by the fungus *M. phaseolina* was air dried and broken down into particles with the aid of a mortar and pestle and passed through a 2 mm mesh sieve.

1 g of the soil passed through the sieve was placed in 2 ml extraction tubes containing glass beads with a diameter of 0.5 mm and every tube contained 1 g of soil. A homogenisation buffer was added to each tube, consisting of: 50 mg of PVP40 and 800 µl of a skimmed milk suspension (3.2%) prepared with sterile water.

The tubes underwent 3 homogenisation cycles with the aid of a bead-beater (BeadBug™ Microtube Homogenizer): each cycle at 4000 rpm lasted 60 seconds and between two successive cycles there were 60 second intervals under ice.

The homogenate was centrifuged at 12000 × *g* for 30 minutes at 4°C to remove the soil.

The supernatant was collected and placed in a 2 ml Eppendorf test tube. Then followed the addition of the lysis buffer consisting of: proteinase K (final concentration 50 µg ml⁻¹) and SDS (final concentration 2%). The suspension obtained was incubated at 65°C for 40 minutes.

At the end of incubation, the lysate was centrifuged at 2500 × *g* for 10 minutes at 4°C and the supernatant was collected.

The volume of the supernatant was measured, adjusting the concentration of NaCl until the final concentration was 1.4 M. Then followed the addition of a 1/10 volume of a CTAB buffer (10% CTAB (cetyltrimethylammonium bromide), 500 mM Tris-HCI, 100 mM EDTA, pH 8.0). The lysate was incubated for 15 minutes at 65°C and cooled for 2 minutes at 15°C.

After cooling, an equal volume of a mixture of chloroform-isoamyl alcohol (24:1) was added to the lysate; the resulting mixture was centrifuged at 6700 × *g* for 10 minutes at 4°C to remove the proteins.

The total nucleic acids were precipitated with the addition, to the aqueous phase recovered from the previous centrifugation, of two volumes of 95% ethanol maintained at 4°C; the resulting mixture was maintained at -20°C for about 30-60 minutes. The sample was kept at -20°C for at least 30 minutes.

The total precipitated nucleic acids were centrifuged at 11600 × *g* for 1 minute at 4°C and the pellet obtained was washed with 70% ethanol, again by centrifuging at the same conditions as described above.

The pellet obtained was dried and dissolved in sterile nuclease-free water. The solution containing the total nucleic acids was maintained at -20°C until the time of use thereof.

### - Development of the species-specific primers for the fungus M. phaseolina for amplifying a gene sequence by PCR

The entire intergenic spacer (IGS) region of the ribosomal DNA of five isolates of the phytopathogenic fungus *Macrophomina phaseolina* was amplified by PCR using the pair of universal primers CNL12/CNS1, which pair, respectively, with the 3' end of the 28S gene and the 5' end of the 18S gene. The sequences obtained from the amplification with the primers CNL12/CNS1 were used to design a pair of specific primers for a more central IGS region. For this purpose, the IGS sequences of various isolates of *M. phaseolina* were aligned with MAFFT software (Katoh and Standley, 2013) in order to identify homologous regions. On the basis of these conserved homologous sequences, the following primers were defined:

| | |
|---|---|
| • MP1F: | 5'- GATTCCCTCCCATCCTCCC -3' |
| • MP1R: | 5'- CCTCGTGTAGCCCGAATTTT -3' |

A second PCR cycle was then carried out using the primers MP1F/ MP1R, which led to the obtainment of an amplification of the central IGS region of the five isolates, with a length of about 1500 bp.

The nucleotide sequences obtained using the primers CNL12/CNS1 and MP1 F/MP1 R were used to design an even more internal primer, by exploiting the above-described technique of alignment and identification of homologous regions. This led to the identification of the primer MP891F (5'-CCG CTC CGC CAT ACA AAT TA-3'), which pairs with a region of about 900 bp at the 3' end of the IGS.

Another PCR cycle was then carried out using the primers MP891F/CNS1, which led to the obtainment of an amplification of a partial IGS region.

A bioinformatics study was subsequently conducted on the genomes available in the GenBank database in order to identify, within the various contigs, some sequences of the ribosomal cluster and of the IGS region of *M. phaseolina* and other fungi belonging to the family *Botryosphaeriaceae.* The sequences obtained by sequencing the IGS region of *M. phaseolina* with MP891F/CNS1 were compared with the sequences identified in the GenBank and relating to other fungi of the family *Botryosphaeriaceae (Diplodia pinea, Diplodia scrobiculata, Diplodia seriata, Neofusicoccum parvum).*

This comparison led to the identification of specific conserved regions of the isolates of *M. phaseolina,* which were used as the basis for designing the pair of primers MP102F/MP102R, which amplifies a region of 102 bp specific for *M. phaseolina.*

| | |
|---|---|
| • MP102F: | 5'- GGTAGTCCCGTACCTGCG -3' |
| • MP102R: | 5'- CAAGTCCGCCGCATGAAAC -3' |

The specificity of the pair of primers MP102F/MP102R was verified on 20 isolates of *M. phaseolina* of different geographical origins and 14 different species of phytopathogenic and saprotrophic fungi commonly present in agricultural soils (*Alternaria brassicicola, Aspergillus* sp., *Botrytis cinerea, Diplodia seriata, Fusarium graminearum, F. oxysporum* f. sp. *basilici, Fusarium solani, Penicillium* sp., *Rhizoctonia solani, Rhizopus* sp., *Sclerotinia sclerotiorum, Sclerotium rolfsii, Trichoderma asperellum, Verticillium dahliae).*

Microsclerotia of *M. phaseolina* were then caused to be produced and they were isolated from the culture soil by sieving with nylon filters having a pore size of 100 µm and 40 µm.

Individual microsclerotia of *M. phaseolina* were manipulated with the aid of an ultrafine brush under a stereomicroscope.

DNA was then extracted from the microsclerotia of *M. phaseolina* (creating four samples in which the DNA was extracted respectively from 1, 10, 100, 200 microsclerotia), both in the absence of soil and inoculated into 1 g of soil.

The DNA of the four above-described samples was amplified using the primer pair specific for *M. phaseolina,* MP102F/MP102R.

### - Diagnosis of the presence of DNA of M. phaseolina by PCR-NALFIA assay.

For the purpose of combining PCR amplification of the DNA of M. *phaseolina* with NALFIA (immunochromatographic assay of the nucleic acids with a lateral flow device) in order to identify the amplification products, the species-specific primers MP102F/MP102R were marked. The forward primer MP102F was marked at the 5' end with biotin and the reverse primer was marked at the 5' end with fluorescein isothiocyanate (FITC).

The pair of marked primers was used to amplify the DNA of all the samples previously described in paragraph 2. The presence of the amplicons marked at their ends with biotin and FITC was detected both by gel electrophoresis and an immunochromatographic assay of the nucleic acids with a lateral flow device (NALFIA) using generic strips produced by companies specialised in the LFD field.

Sensitivity tests were performed on the PCR method using serial dilutions of the target DNA, from 50 ng to 5 fg.

The sensitivity of the NALFIA assay was evaluated using decreasing concentrations of the marked amplicons (120, 96, 48, 24, 12, 6, 1.2, 0.24, 0.048, 0.0096, 0.00192 ng).

The PCR product was purified and its concentration was subsequently evaluated by means of a spectrophotometric reading.

In the light of the foregoing, it has thus been ascertained that the invention completely fulfils the set task, since it provides a method for identifying the presence of DNA of the phytopathogenic fungus *M. phaseolina* in an extremely specific and selective manner. With the method of the invention it is possible to obtain an identification of the presence of genetic material of *M. phaseolina* in short periods of time and even where only a limited sample is available to undergo analysis. Furthermore, the method of the invention makes it possible to identify the presence of *M. phaseolina* directly in soil presumed to be contaminated, especially when the method of the invention is combined with the nucleic acid extraction technique described herein. The method enables the identification of as little 1 microsclerotium per gram of soil, which is a very relevant aspect considering that even a single microsclerotium g⁻¹ soil is capable of causing disease.

Finally, the method according to the invention is economical, efficient and simple to implement.

The method for identifying the presence of DNA of *M. phaseolina* according to the invention, as well as the primers MP102F (SEQ. ID. NO: 4) and MP102R (SEQ. ID. NO: 5) for carrying out PCR according to the method of the invention and the kit comprising said primers, conceived as described herein, are susceptible of numerous modifications and variants, all falling within the scope of the inventive concept; furthermore, all the details may be replaced by other equivalent elements whose correspondence is known to the person skilled in the art.

## Claims

1. A method for determining the presence of Macrophomina phaseolina (M. phaseolina), possibly in the form of a microsclerotium, and/or for detecting the presence of the DNA thereof in a biological sample, comprising the steps of:
(i) Obtaining a biological sample suspected for the presence of M .phaseolina, possibly in the form of a microsclerotium, preferably a biological sample suspected for the presence of DNA of M. phaseolina;
(ii) Amplifying, with at least one pair of oligonucleotides (primers) at least one region of ribosomal DNA (rDNA) of M. phaseolina, said region being SEQ ID NO:3; and
(iii) Detecting the amplified sequence, if present, by means of an analytical method wherein the presence of said amplified sequence signifies the positivity of the sample for the presence of M. phaseolina, preferably for the DNA of M. phaseolina, i.e. that the sample which underwent the method is M .phaseolina-positive.

2. The method according to claim 1, further comprising a step of purifying the nucleic acids, from said biological sample.

3. The method according to claim 1 or 2, wherein said biological sample is selected from among: soil, an infected plant material, preferably leaves, stalk, roots, fruits, or seeds, and crop residues, preferably, the biological sample is a soil sample.

4. The method according to any one of claims 1-3, wherein said pair of oligonucleotides is SEQ ID NO: 4 and 5.

5. The method according to any one of claims 1-4, wherein each primer of the pair is marked at the 5' end with a detectable marker, the marker being preferably selected from among: fluorescein isothiocyanate (FITC), biotin, digoxigenin (DIG), Texas Red® and dinitrophenol (DNP).

6. The method according to any one of claims 1-5, wherein step (iii) is carried out by means of an immunochromatographic assay, preferably with a lateral flow device (NALFIA).

7. Use of SEQ ID NO: 4-5 for determining the presence of M. phaseolina in a sample and/or for detecting and/or identifying the presence of the DNA of M. phaseolina, or for typing M. phaseolina, wherein said M .phaseolina is preferably in the form of a microsclerotium.

8. Kit for determining the presence of M. phaseolina, possibly in the form of a microsclerotium, and/or for detecting the presence of the DNA of M .phaseolina in a biological sample, said kit comprising at least one pair of oligonucleotides (primers) which is SEQ ID NO: 4-5 capable of amplifying at least one region of the ribosomal DNA (rDNA) of M. phaseolina, said region being SEQ ID NO: 3 and optionally reagents suitable for the amplification reaction and/or a positive control, said positive control being at least one region of the ribosomal DNA (rDNA) of M. phaseolina, preferably selected from among SEQ ID NO: 1-3 and/or strips for immunochromatographic detection with a lateral flow device (NALFIA).

9. The kit according to claim 8, wherein each primer of the oligonucleotides pair is marked at the 5' end with a detectable marker, said marker being preferably selected from among: fluorescein isothiocyanate (FITC), biotin, digoxigenin (DIG), Texas Red® and dinitrophenol (DNP).

## Patentansprüche

1. Verfahren zum Bestimmen des Vorhandenseins von Macrophomina phaseolina (M. phaseolina), möglicherweise in Form eines Mikrosklerotiums, und/oder zum Nachweis des Vorhandenseins dessen DNA in einer biologischen Probe, umfassend die Schritte von:
(i) Erhalten einer biologischen Probe, bei der der Verdacht auf das Vorhandensein von M. Phaseolina besteht, möglicherweise in Form eines Mikrosklerotiums, vorzugsweise einer biologischen Probe, bei der der Verdacht auf das Vorhandensein von DNA von M. phaseolina besteht;
(ii) Amplifizieren mit mindestens einem Paar von Oligonukleotiden (Primern) mindestens einer Region ribosomaler DNA (rDNA) von M. phaseolina, wobei die Region SEQ ID NO:3 ist; und
(iii) Nachweis der amplifizierten Sequenz, falls vorhanden, mittels eines Analyseverfahrens, wobei das Vorhandensein der amplifizierten Sequenz für die Positivität der Probe für das Vorhandensein von M. phaseolina, vorzugsweise für die DNA von M. phaseolina, steht, das heißt dass die Probe, die dem Verfahren unterzogen wurde, M-Phaseolina-positiv ist.

2. Verfahren nach Anspruch 1, ferner umfassend einen Schritt zum Purifizieren der Nukleinsäuren aus der biologischen Probe.

3. Verfahren nach Anspruch 1 oder 2, wobei die biologische Probe ausgewählt ist aus: Boden, einem infizierten Pflanzenmaterial, vorzugsweise Blättern, Stielen, Wurzeln, Früchten oder Samen und Ernterückständen, vorzugsweise ist die biologische Probe eine Bodenprobe.

4. Verfahren nach einem der Ansprüche 1-3, wobei das Paar von Oligonukleotiden SEQ ID NO: 4 und 5 ist.

5. Verfahren nach einem der Ansprüche 1-4, wobei ein jeder Primer des Paares am 5'-Ende mit einem nachweisbaren Marker markiert ist, wobei der Marker vorzugsweise ausgewählt ist aus: Fluoresceinisothiocyanat (FITC), Biotin, Digoxigenin (DIG), Texas Red® und Dinitrophenol (DNP).

6. Verfahren nach einem der Ansprüche 1-5, wobei Schritt (iii) mittels eines immunochromatographischen Assays, vorzugsweise mit einer Lateral-Flow-Vorrichtung (NALFIA), durchgeführt wird.

7. Verwendung der SEQ ID NO: 4-5 zum Bestimmen des Vorhandenseins von M. phaseolina in einer Probe und/oder zum Nachweis und/oder Identifizieren des Vorhandenseins der DNA von M. phaseolina oder zum Typisieren von M. phaseolina, wobei das M. phaseolina vorzugsweise in der Form eines Mikrosklerotiums vorliegt.

8. Kit zum Bestimmen des Vorhandenseins von M. phaseolina, möglicherweise in Form eines Mikrosklerotiums, und/oder zum Nachweis des Vorhandenseins der DNA von M. phaseolina in einer biologischen Probe, wobei das Kit mindestens ein Paar von Oligonukleotiden (Primer) umfasst, die SEQ ID NO: 4-5 ist, die in der Lage ist, mindestens eine Region der ribosomalen DNA (rDNA) von M. phaseolina zu amplifizieren, wobei die Region SEQ ID NO: 3 ist und gegebenenfalls Reagenzien, die für die Amplifikationsreaktion und/oder eine positive Kontrolle geeignet sind, wobei die positive Kontrolle mindestens eine Region der ribosomalen DNA (rDNA) von M. phaseolina ist, vorzugsweise ausgewählt aus SEQ ID NO: 1-3 und/oder Streifen zum immunochromatographischen Nachweis mit einem Lateral Flow-Vorrichtung (NALFIA).

9. Kit nach Anspruch 8, wobei ein jeder Primer des Oligonukleotidpaars am 5'-Ende mit einem nachweisbaren Marker markiert ist, wobei der Marker vorzugsweise ausgewählt ist aus: Fluoresceinisothiocyanat (FITC), Biotin, Digoxigenin (DIG), Texas Red® und Dinitrophenol (DNP) .

## Revendications

1. Méthode pour déterminer la présence de Macrophomina phaseolina (M. phaseolina), éventuellement sous la forme d'un microsclérote, et/ou pour détecter la présence de son ADN dans un échantillon biologique, comprenant les étapes de :
(i) Obtenir un échantillon biologique pour lequel l'on soupçonne la présence de M. phaseolina, éventuellement sous la forme d'un microsclérote, de préférence un échantillon biologique pour lequel l'on soupçonne la présence de l'ADN de M. phaseolina ;
(ii) Amplifier, avec au moins une paire d'oligonucléotides (amorces), d'au moins une région de l'ADN ribosomique (ADNr) de M. phaseolina, ladite région étant indiquée dans SEQ ID N° 3 ; et
(iii) Détecter la séquence amplifiée, si elle est présente, au moyen d'une méthode analytique dans laquelle la présence de ladite séquence amplifiée signifie que l'échantillon est positif pour la présence de M. phaseolina, de préférence pour l'ADN de M. phaseolina, c'est-à-dire que l'échantillon qui a subi la méthode est positif au M. phaseolina.

2. Méthode selon la revendication 1, comprenant de plus une étape consistant à purifier les acides nucléiques, à partir dudit échantillon biologique.

3. Méthode selon la revendication 1 ou 2, dans laquelle ledit échantillon biologique est choisi parmi : le sol, un matériau végétal infecté, de préférence des feuilles, des tiges, des racines, des fruits ou des graines, et des résidus de culture, de préférence, l'échantillon biologique est un échantillon de sol.

4. Méthode selon l'une quelconque des revendications 1-3, dans laquelle ladite paire d'oligonucléotides est indiquée dans SEQ ID N° : 4 et 5.

5. Méthode selon l'une quelconque des revendications 1-4, dans laquelle chaque amorce de la paire est marquée à l'extrémité 5' avec un marqueur détectable, le marqueur étant de préférence choisi parmi : l'isothiocyanate de fluorescéine (FITC), la biotine, la digoxigénine (DIG), le Texas Red® et le dinitrophénol (DNP).

6. Méthode selon l'une quelconque des revendications 1-5, dans laquelle l'étape (iii) est réalisée au moyen d'un essai immunochromatographique, de préférence avec un dispositif à flux latéral (NALFIA).

7. Utilisation des SEQ ID N° : 4-5 pour déterminer la présence de M. phaseolina dans un échantillon et/ou pour détecter et/ou identifier la présence de l'ADN de M. phaseolina, ou pour effectuer un typage de M. phaseolina, dans laquelle ledit M. phaseolina est de préférence sous la forme d'un microsclérote.

8. Kit pour déterminer la présence de M. phaseolina, éventuellement sous la forme d'un microsclérote, et/ou pour détecter la présence de l'ADN de M. phaseolina dans un échantillon biologique, ledit kit comprenant au moins une paire d'oligonucléotides (amorces) qui est indiquée dans SEQ ID N° : 4-5 capable d'amplifier au moins une région de l'ADN ribosomique (ADNr) de M. phaseolina, ladite région étant indiquée dans SEQ ID N° : 3 et éventuellement des réactifs adaptés à la réaction d'amplification et/ou à un contrôle positif, ledit contrôle positif étant au moins une région de l'ADN ribosomique (ADNr) de M. phaseolina, de préférence choisie parmi SEQ ID N° : 1-3 et/ou des bandes pour la détection immunochromatographique avec un dispositif à flux latéral (NALFIA).

9. Kit selon la revendication 8, dans lequel chaque amorce de la paire d'oligonucléotides est marquée à l'extrémité 5' avec un marqueur détectable, ledit marqueur étant de préférence choisi parmi : l'isothiocyanate de fluorescéine (FITC), la biotine, la digoxigénine (DIG), le Texas Red® et le dinitrophénol (DNP).
